(19) Europäisches Patentamt
European Patent Office
Office européen des brevets

(11) Numéro de publication : **0 324 692 B1**

(12) # FASCICULE DE BREVET EUROPEEN

(45) Date de publication du fascicule du brevet : **25.11.92 Bulletin 92/48**

(51) Int. Cl.$^5$ : **C07C 303/00,** C07C 309/68, C07C 305/08, C07D 233/91

(21) Numéro de dépôt : **89400096.7**

(22) Date de dépôt : **12.01.89**

(54) **Procédé de préparation d'agents d'hydroxyalkylation, les nouveaux agents ainsi obtenus et leur emploi.**

(30) Priorité : **15.01.88 FR 8800416**

(43) Date de publication de la demande : **19.07.89 Bulletin 89/29**

(45) Mention de la délivrance du brevet : **25.11.92 Bulletin 92/48**

(84) Etats contractants désignés : **AT BE CH DE ES FR GB GR IT LI LU NL SE**

(56) Documents cités : **Eléments de la technique relevés: néant.**

(73) Titulaire : **RHONE-POULENC SANTE**
**20, avenue Raymond Aron**
**F-92160 Antony (FR)**

(72) Inventeur : **Buforn, Albert**
**5 rue Champagneux**
**F-69008 Lyon (FR)**
Inventeur : **Massonneau, Viviane**
**Charrière Blanche Pins 5**
**F-69130 Ecully (FR)**
Inventeur : **Mulhauser, Michel**
**Immeuble Frênes 4 Résidence Charrière Blanche**
**F-69130 Ecully (FR)**

(74) Mandataire : **Pilard, Jacques et al**
**RHONE-POULENC SANTE, Service Brevets,**
**20 Avenue Raymond Aron**
**F-92165 Antony Cédex (FR)**

EP 0 324 692 B1

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention concerne la préparation d'agents d'hydroxyalkylation de formule générale :

$$RCO - O - (\underset{\displaystyle R_1}{\underset{|}{C}}H)_n - O - SO_2 - R_2 \qquad (I)$$

les nouveaux agents ainsi obtenus et leur emploi.

Dans la formule générale (I),
R représente un radical alkyle contenant 1 à 4 atomes de carbone
n est un nombre entier égal à 2 ou 3
les symboles $R_1$, identiques ou différents, représentent un atome d'hydrogène ou un radical alkyle contenant 1 à 4 atomes de carbone, et
$R_2$ représente un radical alkyle contenant 1 à 4 atomes de carbone, phényle ou un radical

$$- O - (\underset{\displaystyle R_1}{\underset{|}{C}}H)_n - O - CO - R$$

dans lequel R, n et $R_1$ sont définis comme précédemment, étant entendu que, dans les deux radicaux

$$- O - (\underset{\displaystyle R_1}{\underset{|}{C}}H)_n - O - CO - R$$

les symboles R, n et $R_1$ ont les mêmes significations.

Plus particulièrement l'invention concerne la préparation des produits de formule générale (I) dans laquelle :
R représente un radical méthyle
n est égal à 2
un des symboles $R_1$ représente un atome d'hydrogène et l'autre représente un atome d'hydrogène ou un radical méthyle, et
$R_2$ représente un radical méthyle ou un radical

$$- O - (\underset{\displaystyle R_1}{\underset{|}{C}}H)_n - O - CO - R$$

dans lequel R représente un radical méthyle, n est égal à 2 et un des symboles $R_1$ représente un atome d'hydrogène et l'autre représente un atome d'hydrogène ou radical méthyle.

Plus particulièrement l'invention concerne la préparation du sulfate de di (acétoxy-2 éthyle) et du mésylate d'acétoxy-2 éthyle.

Selon la présente invention, les produits de formule générale (I) peuvent être obtenus par action d'un acide de formule générale :

$$HO - SO_2 - R_3 \qquad (II)$$

dans laquelle $R_3$ représente un radical hydroxy ou un radical alkyle contenant 1 à 4 atomes de carbone ou phényle sur un diester de formule générale :

$$R - CO - O - \underset{R_1}{\underset{|}{(CH)_n}} - O - CO - R \qquad (III)$$

dans laquelle R, n et $R_1$ sont définis comme précédemment, éventuellement en excès.

Les produits de formule générale (I) dans laquelle $R_2$ représente un radical

$$- O - \underset{R_1}{\underset{|}{(CH)_n}} - O - CO - R$$

peuvent aussi être obtenus par action du sulfate de diméthyle sur un produit de formule générale (III) éventuellement en excès.

Dans tous les cas, la réaction est généralement effectuée par chauffage sous pression réduite (10 à 200 mm de mercure; 1,3 à 26,6 kPa) à une température comprise entre 80 et 160°C en éliminant l'acide formé (RCOOH) ou son ester méthylique par distillation, ainsi, éventuellement, que l'excès d'ester de formule générale (III).

Généralement, le produit de formule générale (I) ainsi obtenu est utilisé sans purification ultérieure.

La présente invention concerne également les produits de formule générale (I) dans laquelle $R_2$ représente un radical

$$- O - \underset{R_1}{\underset{|}{(CH)_n}} - O - CO - R$$

dans lequel n, R et $R_1$ sont définis comme précédemment, et, plus particulièrement le sulfate de di (acétoxy-2 éthyle).

Les produits de formule générale (I) sont particulièrement utiles pour réaliser des réactions d'hydroxyalkylation.

Par exemple, les produits de formule générale (I) peuvent être utilisés pour préparer des hydroxyalkyl-1 nitroimidazoles qui présentent des propriétés thérapeutiques remarquables tels que l'hydroxyéthyl-1 méthyl-2 nitro-5 imidazole (métronidazole) ou l'(hydroxy-2 propyl)-1 méthyl-2 nitro-5 imidazole (secnidazole) ou l'(hydroxy-3 propyl)-1 méthyl-2 nitro-5 imidazole (ternidazole).

Selon la présente invention, les hydroxyalkyl-1 nitroimidazoles peuvent être obtenus par condensation d'un produit de formule générale (I) sur un dérivé de l'imidazole de formule générale :

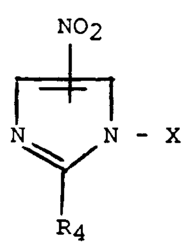

(IV)

dans laquelle $R_4$ représente un atome d'hydrogène ou un radical alkyle contenant 1 à 4 atomes de carbone ou alkényle contenant 2 à 4 atomes de carbone, les radicaux alkyle et alkényle étant éventuellement substitués par un ou plusieurs radicaux, identiques ou différents, choisis parmi les radicaux phényles, phénoxy ou hétérocycliques oxygénés à 5 ou 6 chaînons, ou bien un radical aryle contenant 6 à 10 atomes de carbone éventuellement substitué par un ou plusieurs atomes ou radicaux, identiques ou différents, choisis parmi les atomes d'halogène et les radicaux alkyles contenant 1 à 4 atomes de carbone, alkoxy dont la partie alkyle contient 1 à 4 atomes de carbone, phényle, phénoxy ou nitro, ou bien un radical cycloalkyle contenant 5 ou 6 atomes de carbone, les radicaux phényles, phénoxy ou hétérocycliques pouvant être éventuellement substitués par un ou plusieurs atomes ou radicaux, identiques ou différents, choisis parmi les atomes d'halogène et les radicaux alkyles contenant 1 à 4 atomes de carbone, alkoxy dont la partie alkyle contient 1 à 4 atomes de carbone, phényle, phénoxy ou nitro et X représente un atome d'hydrogène ou un radical hydroxyméthyle, alcoxyméthyle dont la partie alcoyle contient 1 à 4 atomes de carbone ou acyloxyméthyle dont la partie acyle contient 1 à 4 atomes de carbone ou un radical éthylénique allylique, tel que le radical allyle, ou un radical arylméthyle tel que le radical benzyle, suivie de l'hydrolyse ou de l'alcoolyse du produit obtenu.

Généralement, la condensation est effectuée à une température comprise entre 60 et 100°C, de préférence voisine de 80°C, en opérant éventuellement dans un solvant organique choisi parmi les esters (acétate de méthyle, acétate d'éthyle), les éthers (méthyltertiobutyléther), les cétones (méthylisobutylcétone) ou les hydrocarbures aromatiques (toluène, xylène).

Généralement, l'hydrolyse ou l'alcoolyse est effectuée par chauffage dans l'eau ou un alcool (méthanol, éthanol) à une température comprise entre 60 et 100°C.

L'hydroxyalkyl-1 nitro-5 imidazole ainsi obtenu est isolé du mélange réactionnel selon les méthodes habituelles à un pH voisin de 10.

Le dérivé de l'imidazole de formule générale (IV) peut être préparé dans les conditions décrites dans le brevet anglais GB 1 026 631.

Les exemples suivants, donnés à titre non limitatif, illustrent la présente invention.

EXEMPLE 1

Dans un appareil à distiller dont le récepteur est plongé dans un bain acétone - carboglace, on introduit 292,3 g de diacétate de glycol (2 moles) et 50,4 g de sulfate de diméthyle (0,4 mole). On établit dans l'appareil une pression de 200 mm de mercure (26,6 kPa) puis on chauffe le mélange réactionnel pendant 5 heures à 150°C. Pendant le chauffage on distille 60 cm3 d'acétate de méthyle. Le mélange réactionnel est refroidi au voisinage de 100°C puis on distille, sous une pression de 1 mm de mercure (0,13 kPa), 206 cm3 de diacétate de glycol (température du bain : 110°C; température des vapeurs : 65°C).

Dans le bouilleur, on récupère le sulfate de di (acétoxy-2 éthyle) sous forme d'une huile jaune claire.

Le sulfate de di (acétoxy-2 éthyle) est caractérisé par :

- son spectre infra-rouge dont les principales bandes d'absorption caractéristiques exprimées en $cm^{-1}$ sont 1740 (C=O acétate); 1395-1195 ($C-O-SO_2-O-C$) et 1245 (C-O)
- son spectre de résonance magnétique nucléaire du proton (360 MHz; $CD_3CN$; déplacements chimiques en ppm) = 4,45 (t); 4,31 (t) et 2 (s).

Dans un ballon muni d'une agitation magnétique, on introduit 2,7 g de sulfate de di (acétoxy-2 éthyle) (0,01 mole) et 1,99 g d'acétoxyméthyl-1 méthyl-2 nitro-4 imidazole (0,01 mole). Le mélange réactionnel est chauffé à 80°C pendant 5 heures. On ajoute alors 5 cm3 d'eau et on continue le chauffage à 80°C pendant 3 heures.

Après refroidissement, on dose, par chromatographie liquide à haute performance (CLHP), 1,10 g de métronidazole et 0,288 g de méthyl-2 nitro-4 (ou -5) imidazole.

Le taux de transformation de l'acétoxyméthyl-1 méthyl-2 nitro-4 imidazole est de 77,3%.

Le rendement en métronidazole est de 64,2% par rapport à l'acétoxyméthyl-1 méthyl-2 nitro-4 imidazole mis en oeuvre et de 83% par rapport à l'acétoxyméthyl-1 méthyl-2 nitro-4 imidazole transformé.

EXEMPLE 2

Dans un ballon muni d'une agitation magnétique, on introduit 10,8 g de sulfate de di (acétoxy-2 éthyle) (0,04 mole) préparé dans les conditions de l'exemple 1 et 7,96 g d'acétoxyméthyl-1 méthyl-2 nitro-4 imidazole (0,04 mole). Le mélange réactionnel est chauffé à 80°C pendant 4 heures. On ajoute alors 20 cm3 de méthanol et on chauffe à reflux pendant 4 heures. Le dosage de la solution obtenue par CLHP avec étalonnage externe montre qu'elle contient 4,89 g de métronidazole et 0,95 g de méthyl-2 nitro-4 (ou -5) imidazole.

Le taux de transformation de l'acétoxyméthyl-1 méthyl-2 nitro-4 imidazole est de 81%.

Le rendement en métronidazole est de 71,5% par rapport à l'acétoxyméthyl-1 méthyl-2 nitro-4 imidazole mis en oeuvre et de 88% par rapport à l'acétoxyméthyl-1 méthyl-2 nitro-4 imidazole transformé.

EXEMPLE 3

Dans un ballon muni d'un agitateur magnétique, on introduit 7,96 g d'acétoxyméthyl-1 méthyl-2 nitro-4 imidazole (0,04 mole) et 10,8 g de sulfate de di (acétoxy-2 éthyle) (0,04 mole). On chauffe le mélange réactionel à 80°C pendant 4 heures. On ajoute alors 20 cm3 de méthanol et chauffe au reflux pendant 4 heures. Après refroidissement à 20°C, on ajuste le pH à 11,7 par addition de 5,5 cm3 de soude concentrée (d = 1,33). Le précipité qui se forme est séparé par filtration et lavé deux fois par 3 cm3 de méthanol.

Après séchage, on obtient 4,61 g d'un produit dans lequel le mélange des dérivés de l'imidazole est composé de 98,6% de métronidazole et de 0,6% de méthyl-2 nitro-4 (ou -5) imidazole (CLHP avec normalisation interne).

L'analyse du produit obtenu, par CLHP avec étalonnage externe, montre qu'il contient 2,85 g de métronidazole et 0,014 g de méthyl-2 nitro-4 (ou -5) imidazole.

L'analyse du filtrat par CLHP avec étalonnage externe montre qu'il contient 2,15 g de métronidazole et 0,93 g de méthyl-2 nitro-4 (ou -5) imidazole.

Le taux de transformation de l'acétoxmyméthyl-1 méthyl-2 nitro-4 imidazole est de 81,4%.

Le rendement en métronidazole est de 90% par rapport à l'acétoxyméthyl-1 méthyl-2 nitro-4 imidazole transformé.

## EXEMPLE 4

Dans un appareil à distiller dont le récepteur est plongé dans un bain acétone - carboglace, on introdcuit 23,4 g de diacétate de glycol (0,16 mole) et 7,84 g d'acide sulfurique concentré (d = 1,83) (0,08 mole).

On établit une pression de 15 mm de mercure (2 kPa) puis on chauffe le mélange réactionnel pendant 3 heures à 100°C en distillant 8 g d'acide acétique.

Dans le bouilleur, on récupère le sulfate de di (acétoxy-2 éthyle) sous forme d'une huile jaune.

Dans un ballon muni d'une agitation magnétique, on introduit 7 g de sulfate de di (acétoxy-2 éthyle) préparé ci-dessus et 5,1 g d'acétoxyméthyl-1 méthyl-2 nitro-4 imidazole (0,026 mole). On chauffe le mélange réactionnel à 80°C pendant 6 heures. On ajoute alors 13 cm3 d'eau et poursuit le chauffage pendant 4 heures à 90°C.

Dans la solution obtenue, on dose, par CLHP avec étalonnage externe, 2,39 g de métronidazole et 1,03 g de méthyl-2 nitro-4 (ou -5) imidazole.

Le taux de transformation de l'acétoxyméthyl-1 méthyl-2 nitro-4 imidazole est de 68%.

Le rendement en métronidazole est de 54,5% par rapport à l'acétoxyméthyl-1 méthyl-2 nitro-4 imidazole mis en oeuvre et de 79% par rapport à l'acétoxyméthyl-1 méthyl-2 nitro-4 imidazole transformé.

## EXEMPLE 5

Dans un ballon muni d'une agitation magnétique, on introduit 6 g de sulfate de di (acétoxy-2 éthyle) préparé dans les conditions de l'exemple 4 et 4,38 g d'acétoxyméthyl-1 méthyl-2 nitro-4 imidazole (0,022 mole). On chauffe le mélange réactionnel pendant 6 heures à 80°C puis on ajoute 11 cm3 de méthanol et poursuit le chauffage au reflux pendant 4 heures.

Dans la solution obtenue, on dose, par CLHP avec étalonnage externe, 2,47 g de métronidazole et 0,82 g de méthyl-2 nitro-4 (ou -5) imidazole.

Le taux de transformation de l'acétoxyméthyl-1 méthyl-2 nitro-4 imidazole est de 71%.

Le rendement en métronidazole est de 65,6% par rapport à l'acétoxyméthyl-1 méthyl-2 nitro-4 imidazole mis en oeuvre et de 93% par rapport à l'acétoxyméthyl-1 méthyl-2 nitro-4 imidazole transformé.

## EXEMPLE 6

Dans un appareil à distiller dont le récepteur est plongé dans un bain acétone - carboglace, on introduit 9 g de diacétate de glycol (0,062 mole) et 6 g d'acide méthane sulfonique (0,062 mole). On établit dans l'appareil une pression de 15 mm de mercure (2 kPa) puis on chauffe le milieu réactionnel à 110°C pendant 4 heures. Pendant le chauffage, on distille 2,81 g d'acide acétique.

Dans le bouilleur on récupère une huile jaune claire contenant 80% en poids de mésylate d'acétoxyéthylèneglycol.

Le mésylate d'acétoxyéthylèneglycol est caractérisé par :

- son spectre infra-rouge dont les principales bandes d'absorption caractéristiques exprimées en $cm^{-1}$ sont 1740 (C=O acétate), 1360 (C-O) et 1180 ($SO_2$-O)
- son spectre de résonance magnétique nucléaire du proton (360 MHZ; $CD_3CN$, déplacements chimiques en ppm) : 4,36 (t), 4,26 (t), 3,07 (s) et 2 (s).

Dans un ballon muni d'une agitation magnétique, on introduit 2,5 g de mésylate d'acétoxyéthylèneglycol obtenu précédemment et 1,427 g d'acétoxyméthyl-1 méthyl-2 nitro-4 imidazole (0,072 mole). Le mélange réactionnel est chauffé à 90°C pendant 6 heures. On ajoute alors 20 cm3 d'éthanol et on chauffe à reflux pendant 1 heure.

Le dosage de la solution obtenue par chromatographie liquide à haute performance (CLHP) avec étalonnage externe montre qu'elle contient 0,932 g de métronidazole et 0,120 g de méthyl-2 nitro-4 (ou -5) imidazole.

Le taux de transformation de l'acétoxyméthyl-1 méthyl-2 nitro-4 imidazole est de 87%.

Le rendement en métronidazole est de 77% par rapport à l'acétoxyméthyl-1 méthyl-2 nitro-4 imidazole mis en oeuvre et de 89% par rapport à l'acétoxyméthyl-1 méthyl-2 nitro-4 imidazole transformé.

EXEMPLE 7

Dans un ballon muni d'une agitation magnétique, on introduit 2,5 g de mésylate d'acétoxyéthylèneglycol (obtenu selon l'exemple 6) et de 1,405 g d'acétoxyméthyl-1 méthyl-2 nitro-4 imidazole (0,0071 mole). Le mélange réactionnel est chauffé à 90°C pendant 6 heures. On ajoute alors 20 cm3 d'eau et on chauffe à 80°C pendant 1 heure.

Le dosage de la solution obtenue par CLHP avec étalonnage externe montre qu'elle contient 0,907 g de métronidazole et 0,112 g de méthyl-2 nitro-4 (ou -5) imidazole.

Le taux de transformation de l'acétoxyméthyl-1 méthyl-2 nitro-4 imidazole est de 88%.

Le rendement en métronidazole est de 74% par rapport à l'acétoxyméthyl-1 méthoxy-2 nitro-4 imidazole mis en oeuvre et de 85% par rapport à l'acétoxyméthyl-2 nitro-4 imidazole transformé.

EXEMPLE 8

Dans un ballon muni d'une agitation, on introduit 5,4 g de sulfate de di(acétoxy-2 éthyle) et 1,85 g d'acétoxyméthyl-1 nitro-4 imidazole. On ajoute 30 cm3 de xylène puis chauffe le mélange à 80°C pendant 6 heures. On ajoute alors 30 cm3 d'eau puis chauffe le mélange réactionnel au reflux pendant 4 heures.

Le dosage de la phase aqueuse par chromatographie liquide à haute performance (CLHP) avec étalonnage externe montre que :

- le taux de transformation de l'acétoxyméthyl-1 nitro-4 imidazole est de 88%
- le rendement en hydroxyéthyl-1 nitro-5 imidazole est de 97% par rapport à l'acétoxyméthyl-1 nitro-4 imidazole transformé.

## Revendications

### Revendications pour les Etats contractants suivants: AT, BE, CH, DE, FR, GB, IT, LI, LU, NL, SE

**1.** Procédé de préparation d'un agent d'hydroxyalkylation de formule générale :

$$R - CO - O - \underset{\displaystyle R_1}{\underset{|}{(CH)_n}} - O - SO_2 - R_2$$

dans laquelle
R représente un radical alkyle contenant 1 à 4 atomes de carbone,
n est un nombre entier égal à 2 ou 3
les symboles $R_1$, identiques ou différents, représentent un atome d'hydrogène ou un radical alkyle contenant 1 à 4 atomes de carbone, et
$R_2$ représente un radical alkyle contenant 1 à 4 atomes de carbone, phényle ou un radical

$$- O - \underset{\displaystyle R_1}{\underset{|}{(CH)_n}} - O - CO - R$$

dans lequel R, n et $R_1$ sont définis comme précédemment étant entendu que, dans les deux radicaux

$$- O - \underset{\displaystyle R_1}{\underset{|}{(CH)_n}} - O - CO - R$$

les symboles R, n et $R_1$ ont les mêmes significations, caractérisé en ce que l'on fait réagir un acide de formule générale :

$$HO - SO_2 - R_3$$

dans laquelle $R_3$ représente un radical hydroxy ou un radical alkyle contenant 1 à 4 atomes de carbone ou phényle sur un diester de formule générale :

$$R - CO - O - \underset{\substack{| \\ R_1}}{(CH)}_n - O - CO - R$$

dans laquelle R, n et $R_1$ sont définis comme précédemment, à une température comprise entre 80 et 160°C, en éliminant l'acide formé (RCOOH) ou son ester méthylique par distillation sous pression réduite de 1, 3 à 26,6 kPa au fur et à mesure de sa formation, et éventuellement l'excès de diester.

2. Procédé de préparation d'un agent d'hydroxyalkylation de formule générale :

$$R - CO - O\ \underset{\substack{| \\ R_1}}{(CH)}_n - O - SO_2 - O - \underset{\substack{| \\ R_1}}{(CH)}_n - O - CO - R$$

caractérisé en ce que l'on fait réagir le sulfate de diméthyle sur un ester de formule générale :

$$R - CO - O - \underset{\substack{| \\ R_1}}{(CH)}_n - O - CO - R$$

dans laquelle R, n et $R_1$ sont définis comme dans la revendication 1, à une température comprise entre 80 et 160°C en éliminant l'ester méthylique formé ($R\text{-}CO\text{-}O\text{-}CH_3$) par distillation sous pression réduite, et éventuellement l'excès de diester.

3. Procédé de préparation d'un agent d'hydroxyalkylation de formule générale :

$$CH_3 - CO - O - \underset{\substack{| \\ R_1}}{(CH)}_2 - O - SO_2 - R_2$$

dans laquelle $R_2$ représente un radical méthyle ou un radical

$$- O - \underset{\substack{| \\ R_1}}{(CH)}_2 - O - CO - CH_3$$

caractérisé en ce que l'on fait réagir l'acide sulfurique ou l'acide méthanesulfonique sur un diester de formule générale :

$$CH_3 - CO - O - \underset{\substack{| \\ R_1}}{(CH)}_2 - O - CO - CH_3$$

dans laquelle l'un des symboles $R_1$ représente un atome d'hydrogène et l'autre réprésente un atome d'hydrogène ou un radical méthyle, à une température comprise entre 80 et 160°C en éliminant l'acide acétique formé par distillation sous pression réduite.

4. Procédé de préparation d'un agent d'hydroxyalkylation de formule générale :

$$CH_3 - CO - O - \underset{\substack{| \\ R_1}}{(CH)}_2 - O - SO_2 - O - \underset{\substack{| \\ R_1}}{(CH)}_2 - O - CO - CH_3$$

caractérisé en ce que l'on fait réagir le sulfate de diméthyle sur un diester de formule générale :

$$CH_3 - CO - O - \underset{\displaystyle R_1}{\underset{|}{(CH)_2}} - O - CO - CH_3$$

dans laquelle l'un des symboles $R_1$ représente un atome d'hydrogène et l'autre représente un atome d'hydrogène ou un radical méthyle, à une température comprise entre 80 et 160°C en éliminant l'acétate de méthyle formé par distillation sous pression réduite.

5. Procédé de préparation du sulfate de di (acétoxy-2 éthyle) caractérisé en ce que l'on fait réagir l'acide sulfurique ou le sulfate de diméthyle sur le diacétate de glycol à une température comprise entre 80 et 160°C en éliminant l'acide acétique ou l'acétate de méthyle formé par distillation sous pression réduite.

6. Procédé de préparation du mésylate d'acétoxy-2 éthyle caractérisé en ce que l'on fait réagir l'acide méthanesulfonique sur le diacétate du glycol à une température comprise entre 80 et 160°C en éliminant l'acide acétique formé par distillation sous pression réduite.

7. Les nouveaux agents d'hydroxyalkylation de formule générale :

$$R - CO - O \underset{\displaystyle R_1}{\underset{|}{(CH)_n}} - O - SO_2 - O - \underset{\displaystyle R_1}{\underset{|}{(CH)_n}} - O - CO - R$$

dans laquelle
R représente un radical alkyle contenant 1 à 4 atomes de carbone,
n est un nombre entier égal à 2 ou 3, et
les symboles $R_1$, identiques ou différents, représentent un atome d'hydrogène ou un radical alkyle contenant 1 à 4 atomes de carbone.

8. Les nouveaux agents d'hydroxyalkylation de formule générale :

$$CH_3 - CO - O - \underset{\displaystyle R_1}{\underset{|}{(CH)_2}} - O - SO_2 - O - \underset{\displaystyle R_1}{\underset{|}{(CH)_2}} - O - CO - CH_3$$

dans laquelle l'un des symboles $R_1$ représente un atome d'hydrogène ou un radical méthyle.

9. Le sulfate de di (acétoxy-2 éthyle).

10. Procédé de préparation d'un hydroxyalkyl-1 nitro-5 imidazole caractérisé en ce que l'on fait réagir un produit selon la revendication 7 sur un dérivé de l'imidazole de formule générale :

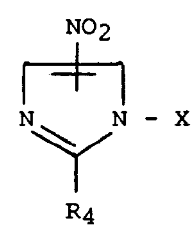

dans laquelle $R_4$ représente un atome d'hydrogène ou un radical alkyle contenant 1 à 4 atomes de carbone ou alkényle contenant 2 à 4 atomes de carbone, les radicaux alkyle et alkényle étant éventuellement substitués par un ou plusieurs radicaux, identiques ou différents, choisis parmi les radicaux phényles, phénoxy ou hétérocycliques oxygénés à 5 ou 6 chaînons, ou bien un radical aryle contenant 6 à 10 atomes de carbone éventuellement substitué par un ou plusieurs atomes ou radicaux, identiques ou différents, choisis parmi les atomes d'halogène et les radicaux alkyles contenant 1 à 4 atomes de carbone, alkoxy dont la partie alkyle contient 1 à 4 atomes de carbone, phényle, phénoxy ou nitro, ou bien un radical cycloalkyle

contenant 5 ou 6 atomes de carbone, les radicaux phényle, phénoxy ou hétérocycliques pouvant être éventuellement substitués par un ou plusieurs atomes ou radicaux, identiques ou différents, choisis parmi les atomes d'halogène et les radicaux alkyles contenant 1 à 4 atomes de carbone, alkoxy dont la partie alkyle contient 1 à 4 atomes de carbone, phényle, phénoxy ou nitro et X représente un atome d'hydrogène ou un radical hydroxyméthyl, alcoxyméthyle dont la partie alcoyle contient 1 à 4 atomes de carbone ou acyloxyméthyle dont la partie acyle contient 1 à 4 atomes de carbone ou un radical éthylénique allylique ou un radical arylméthyle, à une température comprise entre 60 et 100°C en opérant éventuellement dans un solvant organique choisi parmi les esters tels que l'acétate de méthyle ou d'éthyle, les éthers tels que le méthyltertiobutyléther, les cétones telles que la méthylisobutylcétone ou les hydrocarbures aromatiques tels que le toluène ou le xylène, puis soumet le produit obtenu à une hydrolyse ou à un alcoolyse, et isole l'hydroxyalkyl-1 nitro-5 imidazole ainsi obtenu.

**11.** Procédé de préparation d'un hydroxyalkyl-1 nitro-5 imidazole caractérisé en ce que l'on fait réagir un produit selon la revendication 8 sur un dérivé de l'imidazole telle que définie dans la revendication, 10, à une température comprise entre 60 et 100°C en opérant éventuellement dans un solvant organique choisi parmi les esters tels que l'acétate de méthyle ou d'éthyle, les éthers tels que le méthyltertiobutyléther, les cétones telles que la méthylisobutylcétone ou les hydrocarbures aromatiques tels que le toluène ou le xylène, puis soumet le produit obtenu à une hydrolyse ou une alcoolyse, et isole l'hydroxyalkyl-1 nitro-5 imidazole.

**12.** Procédé de préparation de l'hydroxyéthyl-1 méthyl-2 nitro-5 imidazole caractérisé en ce que l'on fait réagir le sulfate de di (acétoxy-2 éthyle) sur un dérivé de l'imidazole de formule générale :

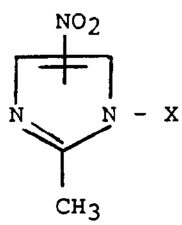

dans laquelle X représente un atome d'hydrogène ou un radical hydroxyméthyl, alcoxyméthyle dont la partie alcoyle contient 1 à 4 atomes de carbone ou acyloxyméthyle dont la partie acyle contient 1 à 4 atomes de carbone ou un radical éthylénique allylique ou un radical aryleméthyle, à une température comprise entre 60 et 100°C en opérant éventuellement dans un solvant organique choisi parmi les esters tels que l'acétate de méthyle ou d'éthyle, les éthers tels que le méthyltertiobutyléther, les cétones telles que la méthylisobutylcétones ou les hydrocarbures aromatiques tels que le toluène ou le xylène, puis soumet le produit obtenu à une hydrolyse ou à une alcoolyse et isole l'hydroxyéthyl-1 méthyl-2 nitro-5 imidazole.

**13.** Procédé de préparation d'un hydroxyalkyl-1 nitro-5 imidazole caractérisé en ce que l'on fait réagir un produit de formule générale :

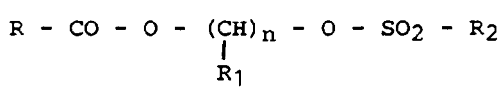

dans laquelle R représente un radical alkyle contenant 1 à 4 atomes de carbone, n est égal à 2 ou 3, les symboles $R_1$, identiques ou différents, représentent un atome d'hydrogène ou un radical alkyle contenant 1 à 4 atomes de carbone et $R_2$ représente un radical alkyle contenant 1 à 4 atomes de carbone ou un radical phényle obtenu selon le procédé de la revendication 1 sur un dérivé de l'imidazole de formule telle que définie dans la revendication 10, à une température comprise entre 60 et 100°C et en opérant éventuellement dans un solvant organique choisi parmi les esters tels que l'acétate de méthyle ou d'éthyle, les éthers tels que le méthyltertiobutyléther les cétones telles que la méthylisobutylcétone ou les hydrocarbures aromatiques tels que le toluène ou le sylène, puis soumet le produit obtenu à une hydrolyse ou à une alcoolyse, et isole l'hydroxyalkyl-1 nitro-5 imidazole.

**14.** Procédé de préparation d'un hydroxyalkyl-1 nitro-5 imidazole caractérisé en ce que l'on fait réagir un pro-

duit de formule générale :

$$CH_3 - CO - O - \underset{R_1}{(CH)_2} - O - SO_2 - R_2$$

dans laquelle $R_2$ représente un radical méthyle et un des symboles $R_1$ représente un atome d'hydrogène et l'autre représente un atome d'hydrogène ou le radical méthyle obtenu selon le procédé de la revendication 3 sur un dérivé de l'imidazole de formule telle que définie dans la revendication 10, à une température comprise entre 60 et 100°C et en opérant éventuellement dans un solvant organique choisi parmi les esters tels que l'acétate de méthyle ou d'éthyle, les éthers tels que le méthyltertiobutyléther, les cétones telles que la méthylisobutylcétone ou les hydrocarbures aromatiques tels que le toluène ou le xylène, puis soumet le produit obtenu à une hydrolyse ou à une alcoolyse, et isole l'hydroxyalkyl-1 nitro-5 imidazole.

**15.** Procédé de préparation de l'hydroxyéthyl-1 méthyl-2 nitro-5 imidazole caractérisé en ce que l'on fait réagir le mésylate d'acétoxy-2 éthyle obtenu selon le procédé de la revendication 6 sur un dérivé de l'imidazole de formule générale :

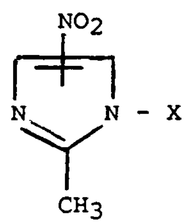

dans laquelle X représente un atome d'hydrogène ou un radical hydroxyméthyle, alcoxyméthyle dont la partie alcoyle contient 1 a 4 atomes de carbone ou acyloxyméthyle dont la partie acyle contient 1 à 4 atomes de carbone ou un radical éthylénique allylique ou un radical arylméthyle, à une température comprise entre 60 et 100°C en opérant éventuellement dans un solvant organique choisi parmi les esters tels que l'acétate de méthyle ou d'éthyle, les éthers tels que le méthyltertiobutyléther, les cétones telles que la méthylisobutylcétone ou les hydrocarbures aromatiques tels que le toluène ou le xylène, puis soumet le produit obtenu à une hydrolyse ou à une alcoolyse, et isole l'hydroxyéthyl-1 méthyl-2 nitro-5 imidazole.

**Revendications pour les Etats contractants suivants: ES, GR**

**1.** Procédé de préparation d'un agent d'hydroxyalkylation de formule générale :

$$R - CO - O - \underset{R_1}{(CH)_n} - O - SO_2 - R_2$$

dans laquelle
R représente un radical alkyle contenant 1 à 4 atomes de carbone,
n est un nombre entier égal à 2 ou 3
les symboles $R_1$, identiques ou différents, représentent un atome d'hydrogène ou un radical alkyle contenant 1 à 4 atomes de carbone,
et
$R_2$ représente un radical alkyle contenant 1 à 4 atomes de carbone, phényle ou un radical

$$- O - \underset{R_1}{(CH)_n} - O - CO - R$$

dans lequel R, n et $R_1$ sont définis comme précédemment étant entendu que, dans les deux radicaux

$$-O-(\underset{R_1}{\underset{|}{C}}H)_n-O-CO-R$$

les symboles R, n et $R_1$ ont les mêmes significations,
caractérisé en ce que l'on fait réagir un acide de formule générale :

$$HO-SO_2-R_3$$

dans laquelle $R_3$ représente un radical hydroxy ou un radical alkyle contenant 1 à 4 atomes de carbone ou phényle sur un diester de formule générale :

$$R-CO-O-(\underset{R_1}{\underset{|}{C}}H)_n-O-CO-R$$

dans laquelle R, n et $R_1$ sont définis comme précédemment, à une température comprise entre 80 et 160°C, en éliminant l'acide formé (RCOOH) ou son ester méthylique par distillation sous pression réduite de 1, 3 à 2 6, 6 kPa au fur et à mesure de sa formation, et éventuellement l'excès de diester.

**2.** Procédé de préparation d'un agent d'hydroxyalkylation de formule générale :

$$R-CO-O\ (\underset{R_1}{\underset{|}{C}}H)_n-O-SO_2-O-(\underset{R_1}{\underset{|}{C}}H)_n-O-CO-R$$

caractérisé en ce que l'on fait réagir le sulfate de diméthyle sur un ester de formule générale :

$$R-CO-O-(\underset{R_1}{\underset{|}{C}}H)_n-O-CO-R$$

dans laquelle R, n et $R_1$ sont définis comme dans la revendication 1, à une température comprise entre 80 et 160°C en éliminant l'ester méthylique formé ($R-CO-O-CH_3$) par distillation sous pression réduite, et éventuellement l'excès de diester.

**3.** Procédé de préparation d'un agent d'hydroxyalkylation de formule générale :

$$CH_3-CO-O-(\underset{R_1}{\underset{|}{C}}H)_2-O-SO_2-R_2$$

dans laquelle $R_2$ représente un radical méthyle ou un radical

$$-O-(\underset{R_1}{\underset{|}{C}}H)_2-O-CO-CH_3$$

caractérisé en ce que l'on fait réagir l'acide sulfurique ou l'acide méthanesulfonique sur un diester de formule générale :

$$CH_3-CO-O-(\underset{R_1}{\underset{|}{C}}H)_2-O-CO-CH_3$$

dans laquelle l'un des symboles $R_1$ représente un atome d'hydrogène et l'autre représente un atome d'hydrogène ou un radical méthyle, à une température comprise entre 80 et 160°C en éliminant l'acide acétique formé par distillation sous pression réduite.

4. Procédé de préparation d'un agent d'hydroxyalkylation de formule générale :

$$CH_3-CO-O-(\underset{R_1}{CH})_2-O-SO_2-O-(\underset{R_1}{CH})_2-O-CO-CH_3$$

caractérisé en ce que l'on fait réagir le sulfate de diméthyle sur un diester de formule générale :

$$CH_3-CO-O-(\underset{R_1}{CH})_2-O-CO-CH_3$$

dans laquelle l'un des symboles $R_1$ représente un atome d'hydrogène et l'autre représente un atome d'hydrogène ou un radical méthyle, à une température comprise entre 80 et 160°C en éliminant l'acétate de méthyle formé par distillation sous pression réduite.

5. Procédé de préparation du sulfate de di (acétoxy-2 éthyle) caractérisé en ce que l'on fait réagir l'acide sulfurique ou le sulfate de diméthyle sur le diacétate de glycol à une température comprise entre 80 et 160°C en éliminant l'acide acétique ou l'acétate de méthyle formé par distillation sous pression réduite.

6. Procédé de préparation du mésylate d'acétoxy-2 éthyle caractérisé en ce que l'on fait réagir l'acide méthanesulfonique sur le diacétate du glycol à une température comprise entre 80 et 160°C en éliminant l'acide acétique formé par distillation sous pression réduite.

7. Utilisation d'un produit de formule générale :

$$R-CO-O\ (\underset{R_1}{CH})_n-O-SO_2-O-(\underset{R_1}{CH})_n-O-CO-R$$

obtenu selon le procédé d'une des revendications 1 ou 2 pour la préparation d'un hydroxyalkyl-1 nitro-5 imidazole caractérisé en ce qu'on le fait réagir sur un dérivé de l'imidazole de formule générale :

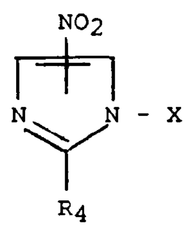

dans laquelle $R_4$ représente un atome d'hydrogène ou un radical alkyle contenant 1 à 4 atomes de carbone ou alkényle contenant 2 à 4 atomes de carbone, les radicaux alkyle et alkényle étant éventuellement substitués par un ou plusieurs radicaux, identiques ou différents, choisis parmi les radicaux phényles, phénoxy ou hétérocycliques oxygénés à 5 ou 6 chaînons, ou bien un radical aryle contenant 6 à 10 atomes de carbone éventuellement substitué par un ou plusieurs atomes ou radicaux, identiques ou différents, choisis parmi les atomes d'halogène et les radicaux alkyles contenant 1 à 4 atomes de carbone, alkoxy dont la partie alkyle contient 1 à 4 atomes de carbone, phényle, phénoxy ou nitro, ou bien un radical cycloalkyle contenant 5 ou 6 atomes de carbone, les radicaux phényle, phénoxy ou hétérocycliques pouvant être éventuellement substitués par un ou plusieurs atomes ou radicaux, identiques ou différents, choisis parmi les atomes d'halogène et les radicaux alkyles contenant 1 à 4 atomes de carbone, alkoxy dont la partie alkyle contient 1 à 4 atomes de carbone, phényle, phénoxy ou nitro et X représente un atome d'hydrogène ou un radical hydroxyméthyl, alcoxyméthyle dont la partie alcoyle contient 1 à 4 atomes de carbone ou acyloxyméthyle dont la partie acyle contient 1 à 4 atomes de carbone ou un radical éthylénique allylique ou un radical arylméthyle, à une température comprise entre 60 et 100°C en opérant éventuellement dans un solvant organique choisi parmi les esters tels que l'acétate de méthyle ou d'éthyle, les éthers tels que le méthyltertiobutyléther, les cétones telles que la méthylisobutylcétone ou les hydrocarbures aromatiques

tels que le toluène ou le xylène, puis soumet le produit obtenu à une hydrolyse ou à un alcoolyse, et isole l'hydroxyalkyl-1 nitro-5 imidazole ainsi obtenu.

**8.** Utilisation d'un produit de formule générale :

$$CH_3 - CO - O - \underset{R_1}{(CH)}_2 - O - SO_2 - O\ \underset{R_1}{(CH)}_2 - O - CO - CH_3$$

obtenu selon le procédé d'une des revendications 3 ou 4 pour la préparation d'un hydroxyalkyl-1 nitro-5 imidazole caractérisé en ce qu'on le fait réagir sur un dérivé de l'imidazole de formule telle que définie dans la revendication 7, à une température comprise entre 60 et 100°C en opérant éventuellement dans un solvant organique choisi parmi les esters tels que l'acétate de méthyle ou d'éthyle, les éthers tels que le méthyltertiobutyléther, les cétones telles que la méthylisobutylcétone ou les hydrocarbures aromatiques tels que le toluène ou le xylène, puis soumet le produit obtenu à une hydrolyse ou à une alcoolyse, et isole l'hydroxyalkyl-1 nitro-5 imidazole.

**9.** Utilisation du sulfate de di(acétoxy-2 éthyle) obtenu selon le procédé de la revendication 5 pour la préparation de l'hydroxyéthyl-1 méthyl-2 nitro-5 imidazole caractérisé en ce qu'on le fait réagir sur un dérivé de l'imidazole de formule générale :

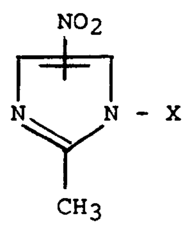

dans laquelle X représente un atome d'hydrogène ou un radical hydroxyméthyl, alcoxyméthyle dont la partie alcoyle contient 1 à 4 atomes de carbone ou acyloxyméthyle dont la partie acyle contient 1 à 4 atomes de carbone ou un radical éthylénique allylique ou un radical aryleméthyle, à une température comprise entre 60 et 100°C en opérant éventuellement dans un solvant organique choisi parmi les esters tels que l'acétate de méthyle ou d'éthyle, les éthers tels que le méthyltertiobutyléther, les cétones telles que la méthylisobutylcétone ou les hydrocarbures aromatiques tels que le toluène ou le xylène, puis soumet le produit obtenu à une hydrolyse ou à une alcoolyse et isole l'hydroxyéthyl-1 méthyl-2 nitro-5 imidazole.

**10.** Utilisation d'un produit de formule générale :

$$R - CO - O - \underset{R_1}{(CH)}_n - O - SO_2 - R_2$$

dans laquelle R représente un radical alkyle contenant 1 à 4 atomes de carbone, n est égal à 2 ou 3, les symboles $R_1$, identiques ou différents, représentent un atome d'hydrogène ou un radical alkyle contenant 1 à 4 atomes de carbone et $R_2$ représente un radical alkyle contenant 1 à 4 atomes de carbone ou un radical phényle obtenu selon le procédé de la revendication 1 pour la préparation d'un hydroxyalkyl-1 nitro-5 imidazole caractérisé en ce qu'on le fait réagir sur un dérivé de l'imidazole de formule telle que définie dans la revendication 7, à une température comprise entre 60 et 100°C et en opérant éventuellement dans un solvant organique choisi parmi les esters tels que l'acétate de méthyle ou d'éthyle, les éthers tels que le méthyltertiobutyléther, les cétones telles que la méthylisobutylcétone ou les hydrocarbures aromatiques tels que le toluène ou le xylène, puis soumet le produit obtenu à une hydrolyse ou à une alcoolyse, et isole l'hydroxyalkyl-1 nitro-5 imidazole.

**11.** Utilisation d'un produit de formule générale :

$$CH_3 - CO - O - \underset{R_1}{(CH)}_2 - O - SO_2 - R_2$$

dans laquelle $R_2$ représente un radical méthyle et un des symboles $R_1$ représente un atome d'hydrogène et l'autre représente un atome d'hydrogène ou le radical méthyle obtenu selon le procédé de la revendication 3 pour la préparation d'un hydroxyalkyl-1 nitro-5 imidazole caractérisé en ce ce qu'on le fait réagir sur un dérivé de l'imidazole de formule telle que définie dans la revendication 7, à une température comprise entre 60 et 100°C et en opérant éventuellement dans un solvant organique choisi parmi les esters tels que l'acétate de méthyle ou d'éthyle, les éthers tels que le méthyltertiobutyléther, les cétones telles que la méthylisobutylcétone ou les hydrocarbures aromatiques tels que le toluène ou le xylène, puis soumet le produit obtenu à une hydrolyse ou à une alcoolyse, et isole l'hydroxyalkyl-1 nitro-5 imidazole.

**12.** Utilisation du mésylate d'acétoxy-2 éthyle obtenu selon le procédé de la revendication 6 pour la préparation de l'hydroxyéthyl-1 méthyl-2 nitro-5 imidazole caractérisé en ce qu'on le fait réagir sur un dérivé de l'imidazole de formule générale :

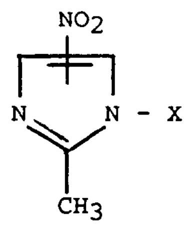

dans laquelle X représente un atome d'hydrogène ou un radical hydroxyméthyle, alcoxyméthyle dont la partie alcoyle contient 1 à 4 atomes de carbone ou acyloxyméthyle dont la partie acyle contient 1 à 4 atomes de carbone ou un radical éthylénique allylique ou un radical arylméthyle, à une température comprise entre 60 et 100°C en opérant éventuellement dans un solvant organique choisi parmi les esters tels que l'acétate de méthyle ou d'éthyle, les éthers tels que le méthyltertiobutyléther, les cétones telles que la méthylisobutylcétone ou les hydrocarbures aromatiques tels que le toluène ou le xylène, puis soumet le produit obtenu à une hydrolyse ou à une alcoolyse, et isole l'hydroxyéthyl-1 méthyl-2 nitro-5 imidazole.

## Patentansprüche

### Patentansprüche für folgende Vertragsstaaten: AT, BE, CH, DE, FR, GB, IT, LI, LU, NL, SE

**1.** Verfahren zur Herstellung eines Hydroxyalkylierungsmittels der allgemeinen Formel

$$R - CO - O - \underset{R_1}{(CH)}_n - O - SO_2 - R_2$$

worin

R einen Alkylrest mit 1 bis 4 Kohlenstoffatomen bedeutet,

n eine ganze Zahl entsprechend 2 oder 3 ist,

die Symbole $R_1$, die gleich oder voneinander verschieden sind, für ein Wasserstoffatom oder einen Alkylrest mit 1 bis 4 Kohlenstoffatomen stehen, und

$R_2$ einen Alkylrest mit 1 bis 4 Kohlenstoffatomen, Phenyl oder einen Rest

$$- O - \underset{R_1}{(CH)}_n - O - CO - R$$

worin R, n und $R_1$ wie vorstehend definiert sind, bedeutetet mit der Maßgabe, daß in den beiden Resten

$$-O-(\underset{R_1}{CH})_n-O-CO-R$$

die Symbole R, n und $R_1$ die gleichen Bedeutungen besitzen, dadurch gekennzeichnet, daß man eine Säure der allgemeinen Formel

$$HO-SO_2-R_3$$

worin $R_3$ für eine Hydroxygruppe oder einen Alkylrest mit 1 bis 4 Kohlenstoffatomen oder Phenyl steht, mit einem Diester der allgemeinen Formel

$$R-CO-O-(\underset{R_1}{CH})_n-O-CO-R$$

worin R, n und $R_1$ wie vorstehend definiert sind, bei einer Temperatur zwischen 80 und 160°C unter Entfernung der gebildeten Säure (RCOOH) oder ihres Methylesters durch Destillation unter vermindertem Druck von 1,3 bis 26,6 kPa nach Maßgabe ihrer Bildung und gegebenenfalls von überschüssigem Diester umsetzt.

**2.** Verfahren zur Herstellung eines Hydroxyalkylierungsmittels der allgemeinen Formel

$$R-CO-O\ (\underset{R_1}{CH})_n-O-SO_2-O-(\underset{R_1}{CH})_n-O-CO-R$$

dadurch gekennzeichnet, daß man Dimethylsulfat mit einem Ester der allgemeinen Formel

$$R-CO-O-(\underset{R_1}{CH})_n-O-CO-R$$

worin R, n und $R_1$ wie in Anspruch 1 definiert sind, bei einer Temperatur zwischen 80 und 160°C und unter Entfernung des gebildeten Methylesters ($R-CO-O-CH_3$) durch Destillation unter vermindertem Druck und gegebenenfalls von überschüssigem Diester umsetzt.

**3.** Verfahren zur Herstellung eines Hydroxyalkylierungsmittels der allgemeinen Formel

$$CH_3-CO-O-(\underset{R_1}{CH})_2-O-SO_2-R_2$$

worin $R_2$ für einen Methylrest oder einen Rest

$$-O-(\underset{R_1}{CH})_2-O-CO-CH_3$$

steht, dadruch gekennzeichnet, daß man Schwefelsäure oder Methansulfonsäure mit einem Diester der allgemeinen Formel

$$CH_3 - CO - O - \underset{R_1}{(\overset{|}{C}H)}_2 - O - CO - CH_3$$

worin eines der Symbole $R_1$ für ein Wasserstoffatom und das andere für ein Wasserstoffatom oder einen Methylrest steht, bei einer Temperatur zwischen 80 und 160°C unter Entfernung der gebildeten Essigsäure durch Destillation unter vermindertem Druck umsetzt.

4. Verfahren zur Herstellung eines Hydroxyalkylierungsmittels der allgemeinen Formel

$$CH_3 - CO - O - \underset{R_1}{(\overset{|}{C}H)}_2 - O - SO_2 - O - \underset{R_1}{(\overset{|}{C}H)}_2 - O - CO - CH_3$$

dadurch gekennzeichnet, daß man Dimethylsulfat mit einem Diester der allgemeinen Formel

$$CH_3 - CO - O - \underset{R_1}{(\overset{|}{C}H)}_2 - O - CO - CH_3$$

worin eines der Symbole $R_1$ für ein Wasserstoffatom und das andere für ein Wasserstoffatom oder einen Methylrest steht, bei einer Temperature zwischen 80 und 160°C unter Entfernung von gebildetem Methylacetat durch Destillation unter vermindertem Druck umsetzt.

5. Verfahren zur Herstellung von Di-(2-acetoxyethyl)-sulfat, dadurch gekennzeichnet, daß man Schwefelsäure oder Dimethylsulfat mit Glykoldiacetat bei einer Temperatur zwischen 80 und 160°C unter Entfernung der gebildeten Essigsäure oder des gebildeten Methylacetats durch Destillation unter vermindertem Druck umsetzt.

6. Verfahren zur Herstellung von 2-Acetoxyethylmesylat, dadurch gekennzeichnet, daß man Methansulfonsäure mit Glykoldiacetat bei einer Temperatur zwischen 80 und 160°C unter Entfernung der gebildeten Essigsäure durch, Destillation unter vermindertem Druck umsetzt.

7. Neue Hydroxyalkylierungsnittel der allgemeinen Formel

$$R - CO - O\ \underset{R_1}{(\overset{|}{C}H)}_n - O - SO_2 - O - \underset{R_1}{(\overset{|}{C}H)}_n - O - CO - R$$

worin R für einen Alkylrest mit 1 bis 4 Kohlenstoffatomen steht, n eine ganze Zahl entsprechend 2 oder 3 ist und die Symbole $R_1$, die identisch oder voneinander verschieden sind, ein Wasserstoffatom oder einen Alkylrest mit 1 bis 4 Kohlenstoffatomen bedeuten.

8. Neue Hydroxyalkylierungsmittel der allgemeinen Formel

$$CH_3 - CO - O - \underset{R_1}{(\overset{|}{C}H)}_2 - O - SO_2 - O - \underset{R_1}{(\overset{|}{C}H)}_2 - O - CO-CH_3$$

worin eines der Symbole $R_1$ für ein Wasserstoffatom oder einen Methylrest steht.

9. Di-(2-acetoxyethyl)-sulfat.

10. Verfahren zur Herstellung eines 1-Hydroalkyl-5-nitroimidazols, dadurch gekennzeichnet, daß man ein Produkt gemäß Anspruch 7 mit einem Imidazolderivat der allgemeinen Formel

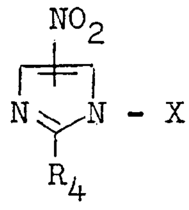

worin $R_4$ ein Wasserstoffatom oder einen Alkylrest mit 1 bis 4 Kohlenstoffatomen oder einen Alkenylrest mit 2 bis 4 Kohlenstoffatomen, wobei die Alkyl- und Alkenylreste gegebenenfalls durch einen oder mehrere identische oder vonenander verschiedene Reste substituiert sind, ausgewählt under der Phenyl-, Phenoxy- oder sauerstoffhaltigen heterocyclischen Resten mit 5 oder 6 Kettengliedern, oder einen Arylrest mit 6 bis 10 Kohlenstoffatomen, gegebenenfalls substituiert durch ein oder mehrere identische oder voneinander verschiedene Atome oder Reste, ausgewählt unter den Halogenatomen und den Alkylresten mit 1 bid 4 Kohlenstoffatomen, den Alkoxyresten, deren Alkylteil 1 bis 4 Kohlenstoffatome aufweist, Phenyl, Phenoxy oder Nitro, oder einen Cycloalkylrest mit 5 oder 6 Kohlenstoffatomen, wobei die Phenyl-, Phenoxy- oder heterocyclischen Reste gegebenenfalls substituiert sein können durch ein oder mehrere identische oder voneinander verschiedene Atome oder Reste, ausgewählt unter den Halogenatomen und den Alkylresten mit 1 bis 4 Kohlenstoffatomen, den Alkoxyresten, deren Alkylteil 1 bis 4 Kohlenstoffatome enthält, der Phenyl-, Phenoxy- oder Nitrogruppe, bedeutet und X ein Wasserstoffatom oder einen Hydroxymethylrest, einen Alkoxymethylrest, dessen Alkylteil 1 bis 4 Kohlenstoffatome enthält, oder einen Acyloxymethylrest, dessen Acylteil 1 bis 4 Kohlenstoffatome enthält, oder einen allylischen, ethylenischen Rest oder einen Arylmethylrest wiedergibt, bei einer Temperatur zwischen 60 und 100° umsetzt, wobei man gegebenenfalls in einem organischen Lösungsmittel, ausgewählt unter den Estern, wie Methyl- oder Ehtylacetat, den Ethern, wie Methyl-tert.-butylether, den Ketonen, wie Methylisobutylketon, oder den aromatischen Kohlenwasserstoffen, wie Toluol oder Xylol, arbeitet, und hiernach das erhaltene Produkt einer Hydrolyse oder einer Alkoholyse unterwirft und das so erhaltene 1-Hydroxyalkyl-5-nitroimidazol isoliert.

**11.** Verfahren zur Herstellung eines 1-Hydroxyalkyl-5-nitroimidazols, dadurch gekennzeichnet, daß man ein Produkt gemäß Anspruch 8 mit einem Imidazolderivat, wie in Anspruch 10 definiert, bei einer Temperatur zwischen 60 und 100°C umsetzt, wobei man gegebenenfalls in einem organischen Lösungsmittel, ausgewählt unter den Estern, wie Methyl- oder Ethylacetat, den Ethern, wie Methyl-tert.-butylether, den Ketonen, wie Methylisobutylketon, oder den aromatischen Kohlenwasserstoffen, wie Toluol oder Xylol, arbeitet, danach das erhaltene Produkt einer Hydrolyse oder einer Alkoholyse unterwirft und das 1-Hydroxyalkyl-5-nitroimidazol isoliert.

**12.** Verfahren zur Herstellung von 1-Hydroxyethyl-2-methyl-5-nitroimidazol, dadurch gekennzeichnet, daß man Di-(2-acetoxyethyl)-sulfat mit einem Imidazolderivat der allgemeinen Formel

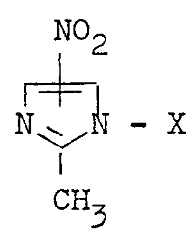

worin X für ein Wasserstoffatom oder einen Hydroxymethyl-rest, einen Alkoxymethylrest, dessen Alkylteil 1 bis 4 Kohlenstoffatome enthält, oder einen Acyloxymethylrest, dessen Acylteil 1 bis 4 Kohlenstoffatome enthält, oder einen allylischen, ethylenischen Rest oder einen Arylmethylrest steht, bei einer Temperatur zwischen 60 und 100°C umsetzt, wobei man gegebenenfalls in einem organischen Lösungsmittel, ausgewählt unter den Estern, wie Methyloder Ethylacetat, den Ethern, wie Methyl-tert.-butylether, den Ketonen, wie Mehtylisobutylketon, oder den aromatischen Kohlenwasserstoffen, wie Toluol oder Xylol, arbeitet, anschließend das erhaltene Produkt einer Hydrolyse oder einer Alkoholyse unterzieht und das 1-Hydroxyethyl-2-methyl-5-nitroimidazol isoliert.

**13.** Verfahren zur Herstellung eines 1-Hydroxyalkyl-5-nitroimidazols, dadurch gekennzeichnet, daß man ein Produkt der allgemeinen Formel

$$R - CO - O - (\underset{R_1}{CH})_n - O - SO_2 - R_2$$

worin R für einen Alkylrest mit 1 bis 4 Kohlenstoffatomen steht, n 2 oder 3 bedeutet, die Symbole $R_1$, die identisch oder voneinander verschieden sind, ein Wasserstoffatom oder einen Alkylrest mit 1 bis 4 Kohlenstoffatomen wiedergeben und $R_2$ einen Alkylrest mit 1 bis 4 Kohlenstoffatomen oder einen Phenylrest bedeutet, erhalten gemäß dem Verfahren nach Anspruch 1, mit einem Imidazolderivat der Formel, wie in Anspruch 10 definiert, bei einer Temperatur zwischen 60 und 100°C umsetzt, wobei man gegebenenfalls in einem organischen Lösungsmittel, ausgewählt unter den Estern, wie Methyl- oder Ethylacetat, den Ethern, wie Methyl-tert.-butylether, den Ketonen, wie Methylisobutylketon, oder den aromatischen Kohlenwasserstoffen, wie Toluol oder Xylol, arbeitet, danach das erhaltene Produkt einer Hydrolyse oder Alkoholyse unterzieht und das 1-Hydroxyalkyl-5-nitroimidazol isoliert.

**14.** Verfahren zur Herstellung eines 1-Hydroxyalkyl-5-nitroimidazols, dadurch gekennzeichnet, daß man ein Produkt der allgemeinen Formel

$$CH_3 - CO - O - (\underset{R_1}{CH})_2 - O - SO_2 - R_2$$

worin $R_2$ für einen Methylrest steht und eines der Symbole $R_1$ ein Wasserstoffatom und das andere ein Wasserstoffatom oder den Methylrest wiedergibt, erhalten nach dem Verfahren des Anspruchs 3, mit einem Imidazolderivat der Formel, wie in Anspruch 10 definiert, bei einer Temperatur zwischen 60 und 100°C umsetzt, wobei man gegebenenfalls in einem organischen Lösungsmittel, ausgewählt unter den Estern, wie Methyl- oder Ethylacetat, den Ethern, wie Methyl-tert.-butylether, den Ketonen, wie Methylisobutyl-keton, oder den aromatischen Kohlenwasserstoffen, wie Toluol oder Xylol, arbeitet, danach das erhaltene Produkt einer Hydrolyse oder Alkoholyse unterzieht und das 1-Hydroxyalkyl-5-nitroimidazol isoliert.

**15.** Verfahren zur Herstellung von 1-Hydroxyethyl-2-methyl-5-nitroimidazol, dadurch gekennzeichnet, daß man 2-Acetoxyethylmesylat, erhalten gemäß dem Verfahren nach Anspruch 6, mit einem Imidazolderivat der allgemeinen Formel

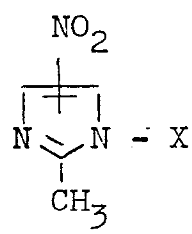

worin X für ein Wasserstoffatom oder einen Hydroxymethyl-rest, einen Alkoxymethylrest, dessen Alkylteil 1 bis 4 Kohlenstoffatome enthält, oder einen Acylosymethylrest, dessen Acylteil 1 bis 4 Kohlenstoffatome enthält, oder einen allylischen, ethylenischen Rest oder einen Arylmethylrest bedeutet, bei einer Temperatur zwischen 60 und 100°C umsetzt, wobei man gegebenenfalls in einen organischen Lösungsmittel, ausgewählt unter den Estern, wie Methyl- oder Ethylacetat, den Ethern, wie Methyl-tert.-butylether, den Ketonen, wie Methylisobutylketon, oder den aromatischen Kohlenwasserstoffen, wie Toluol oder Xylol, arbeitet, danach das erhaltene Produkt einer Hydrolyse oder Alkoholyse unterwirft und das 1-Hydroxyethyl-2-methyl-5-nitroimidazol isoliert.

**Patentansprüche für folgende Vertragsstaaten: ES, GR,**

**1.** Verfahren zur Herstellung eines Hydroxyalkylierungsmittels der allgemeinen Formel

$$R - CO - O - (\underset{R_1}{CH})_n - O - SO_2 - R_2$$

worin

R einen Alkylrest mit 1 bis 4 Kohlenstoffatomen bedeutet,

n eine ganze Zahl entsprechend 2 oder 3 ist,

die Symbole $R_1$, die gleich oder voneinander verschieden sind, für ein Wasserstoffatom oder einen Alkylrest mit 1 bis 4 Kohlenstoffatomen stehen, und

R2 einen Alkylrest mit 1 bis 4 Kohlenstoffatomen, Phenyl oder einen Rest

$$- O - (\underset{R_1}{CH})_n - O - CO - R$$

worin R, n und $R_1$ wie vorstehend definiert sind, bedeutet, mit der Maßgabe, daß in den beiden Resten

$$- O - (\underset{R_1}{CH})_n - O - CO - R$$

die Symbole R, n und $R_1$ die gleichen Bedeutungen besitzen, dadurch gekennzeichnet, daß man eine Säure der allgemeinen Formel

$$HO - SO_2 - R_3$$

worin $R_3$ für eine Hydroxygruppe oder einen Alkylrest mit 1 bis Kohlenstoffatomen oder Phenyl steht, mit einem Diester der allgemeinen Formel

$$R - CO - O - (\underset{R_1}{CH})_n - O - CO - R$$

worin R, n und $R_1$ wie vorstehend definiert sind, bei einer Temperatur zwischen 80 und 160°C unter Entfernung der gebildeten Säure (RCOOH) oder ihres Methylesters durch Destillation unter vermindertem Druck von 1,3 bis 26,6 kPa nach Maßgabe ihrer Bildung und gegebenenfalls von überschüssigem Diester umsetzt.

**2.** Verfahren zur Herstellung eines Hydroxyalkylierungsmittels der allgemeinen Formel

$$R - CO - O\ (\underset{R_1}{CH})_n - O - SO_2 - O - (\underset{R_1}{CH})_n - O - CO - R$$

dadurch gekennzeichnet, daß man Dimethylsulfat mit einem Ester der allgemeinen Formel

$$R - CO - O - (\underset{R_1}{CH})_n - O - CO - R$$

worin R, n und $R_1$ wie in Anspruch 1 definiert sind, bei einer Temperatur zwischen 80 und 160°C und unter Entfernung des gebildeten Methylesters ($R-CO-O-CH_3$) durch Destillation unter vermindertem Druck und gegebenenfalls von überschüssigem Diester umsetzt.

3. Verfahren zur Herstellung eines Hydroxyalkylierungsmittels der allgemeinen Formel

$$CH_3 - CO - O - (\underset{R_1}{CH})_2 - O - SO_2 - R_2$$

worin $R_2$ für einen Methylrest oder einen Rest

$$- O - (\underset{R_1}{CH})_2 - O - CO - CH_3$$

steht, dadurch gekennzeichnet, daß man Schwefelsäure oder Methansulfonsäure mit einem Diester der allgemeinen Formel

$$CH_3 - CO - O - (\underset{R_1}{CH})_2 - O - CO - CH_3$$

worin eines der Symbole $R_1$ für ein Wasserstoffatom und das andere für ein Wasserstoffatom oder einen Methylrest steht, bei einer Temperatur zwischen 80 und 160°C unter Entfernung der gebildeten Essigsäure durch Destillation unter vermindertem Druck umsetzt.

4. Verfahren zur Herstellung eines Hydroxyalkylie-rungsmittels der allgemeinen Formel

$$CH_3 - CO - O - (\underset{R_1}{CH})_2 - O - SO_2 - O - (\underset{R_1}{CH})_2 - O - CO - CH_3$$

dadurch gekennzeichnet, daß man Dimethylsulfat mit einem Diester der allgemeinen Formel

$$CH_3 - CO - O - (\underset{R_1}{CH})_2 - O - CO - CH_3$$

worin eines der Symbole $R_1$ für ein Wasserstoffatom und das andere für ein Wasserstoffatom oder einen Methylrest steht, bei einer Temperatur zwischen 80 und 160°C unter Entfernung von gebildetem Methylacetat durch Destillation unter vermindertem Druck umsetzt.

5. Verfahren zur Herstellung von Di-(2-acetoxyethyl)-sulfat, dadurch gekennzeichnet, daß man Schwefelsäure oder Dimethylsulfat mit Glykoldiacetat bei einer Temperatur zwischen 80 und 160°C unter Entfernung der gebildeten Essigsäure oder des gebildeten Methylacetats durch Destillation unter vermindertem Druck umsetzt.

6. Verfahren zur Herstellung von 2-Acetoxyethylmesylat, dadurch gekennzeichnet, daß man Methansulfonsäure mit Glykoldiacetat bei einer Temperatur zwischen 80 und 160°C unter Entfernung der gebildeten Essigsäure durch Destillation unter vermindertem Druck umsetzt.

7. Verwendung eines Produkts der allgemeinen Formel

$$R - CO - O\ (\underset{R_1}{CH})_n - O - SO_2 - O - (\underset{R_1}{CH})_n - O - CO - R$$

erhalten nach dem Verfahren eines der Ansprüche 1 oder 2, zur Herstellung eines 1-Hydroxyalkyl-5-nitroimidazols, dadurch gekennzeichnet, daß man es mit einem Imidazol-derivat der allgemeinen Formel

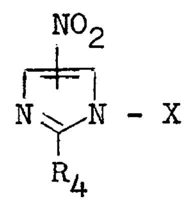

worin $R_4$ ein Wasserstoffatom oder einen Alkylrest mit 1 bis 4 Kohlenstoffatomen oder einen Alkenylrest mit 2 bis 4 Kohlenstoffatomen, wobei die Alkyl- und Alkenylreste gegebenenfalls durch einen oder mehrere identische oder von einander verschiedene Reste substituiert sind, ausgewählt unter den Phenyl-, Phenoxy- oder sauerstoffhaltigen heterocyclischen Resten mit 5 oder 6 Kettengliedern, oder einen Arylrest mit 6 bis 10 Kohlenstoffatomen, gegebenenfalls substituiert durch ein oder mehrere identische oder von einander verschiedene Atome oder Reste, ausgewählt unter den Halogenatomen und den Alkylresten mit 1 bis 4 Kohlenstoffatomen, den Alkoxyresten, deren Alkylteil 1 bis 4 Kohlenstoffatome aufweist, Phenyl, Phenoxy oder Nitro, oder einen Cycloalkylrest mit 5 oder 6 Kohlenstoffatomen, wobei die Phenyl-, Phenoxy- oder heterocyclischen Reste gegebenenfalls substituiert sein können durch ein oder mehrere identische oder voneinander verschiedene Atome oder Reste, ausgewählt unter den Halogenatomen und den Alkylresten mit 1 bis 4 Kohlenstoffatomen, den Alkoxy-resten, deren Alkylteil 1 bis 4 Kohlenstoffatome enthält, der Phenyl-, Phenoxy- oder Nitrogruppe, bedeutet und X ein Wasserstoffatom oder einen Hydroxyethylrest, einen Alkoxymethylrest, dessen Alkylteil 1 bis 4 Kohlenstoffatome enthält, oder einen Acyloxymethylrest, dessen Acylteil 1 bis 4 Kohlenstoffatome enthält, oder einen allylischen, ethylenischen Rest oder einen Arylmethylrest wiedergibt, bei einer Temperatur zwischen 60 und 100°C umsetzt, wobei man gegebenenfalls in einem organischen Lösungsmittel, ausgewählt unter den Estern, wie Methyl- oder Ethylacetat, den Ethern, wie Methyl-tert.-butylether, den Ketonen, wie Methylisobutylketon, oder den aromatischen Kohlenwasserstoffen, wie Toluol oder Xylol, arbeitet, und hiernach das erhaltene Produkt einer Hydrolyse oder einer Alkoholyse unterwirft und das so erhaltene 1-Hydro-xyalkyl-5-nitroimidazol isoliert.

**8.** Verwendung eines Produkts der allgemeinen Formel

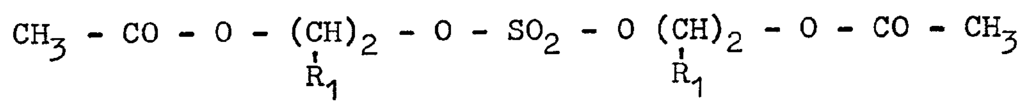

erhalten gemäß den Verfahren eines der Ansprüche 3 oder 4, zur Herstellung eines 1-Hydroxyalkyl-5-nitroimidazols, dadurch gekennzeichnet, daß man es mit einem Imidazol-derivat der Formel, wie in Anspruch 7 definiert, bei einer Temperatur zwischen 60 und 100°C ansetzt, wobei man gegebenenfalls in einem organischen Lösungsmittel, ausgewählt unter den Estern, wie Methyl- oder Ethylacetat, den Ethern, wie Methyl-tert.-butylether, den Ketonen, wie Methylisobutylketon, oder den aromatischen Kohlenwasser-stoffen, wie Toluol oder Xylol, arbeitet, danach das erhaltene Produkt einer Hydrolyse oder einer Alkoholyse untewirft und das 1-Hydroxyalkyl-5-nitroimidazol isoliert.

**9.** Verwendung von Di-(2-acetoxyethyl)-sulfat, erhalten gemäß dem Verfahren des Anspruchs 5, zur Herstellung von 1-Hydroxyethyl-2-methyl-5-nitroimidazol, dadurch gekennzeichnet, daß man es mit einem Imidazolderivat der allgemeinen Formel

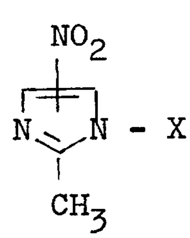

worin X für ein Wasserstoffatom oder einen Hydroxymethylrest, einen Alkoxymethylrest, dessen Alkylteil 1 bis 4 Kohlenstoffatome enthält, oder einen Acyloxymethylrest, dessen Acylteil 1 bis 4 Kohlenstoffatome enthält, oder einen allylischen, ethylenischen Rest oder einen Arylmethylrest steht, bei einer Temperatur zwischen 60 und 100°C umsetzt, wobei man gegebenenfalls in einem organischen Lösungsmittel, ausgewählt unter den Estern, wie Methyl- oder Ethylacetat, den Ethern, wie Methyl-tert.-butylether, den Ketonen, wie Methylisobutylketon, oder den aromatischen Kohlenwasserstoffen, wie Toluol oder Xylol, arbeitet, anschließend das erhaltene Produkt einer Hydrolyse oder einer Alkoholyse unterzieht und das 1-Hydroxyethyl-2-methyl-2-nitroimidazol isoliert.

**10.** Verwendung eines Produkts der allgemeinen Formel

$$R - CO - O - (\underset{R_1}{CH})_n - O - SO_2 - R_2$$

worin R für einen Alkylrest mit 1 bis 4 Kohlenstoffatomen steht, n 2 oder 3 bedeutet, die Symbole $R_1$, die identisch oder voneinander verschieden sind, ein Wasserstoffatom oder einen Alkylrest mit 1 bis 4 Kohlenstoffatomen wiedergeben und $R_2$ einen Alkylrest mit 1 bis 4 Kohlenstoffatomen oder einen Phenylrest bedeutet, erhalten gemäß dem Verfahren nach Anspruch 1, zur Herstellung eines 1-Hydroxyalkyl-5-nitroimidazols, dadurch gekennzeichnet, daß man es mit einem Imidazolderivat der Formel, wie in Anspruch 7 definiert, bei einer Temperatur zwischen 60 und 100°C umsetzt, wobei man gegebenenfalls in einem organischen Lösungsmittel, ausgewählt unter den Estern, wie Methyl- oder Ethylacetat, den Ethern, wie Methyl-tert.-butylether, den betonen, wie Methylisobutylketon, oder den aromatischen Kohlenwasserstoffen, wie Toluol oder Xylol, arbeitet, danach das erhaltene Produkt einer Hydrolyse oder einer Alkoholyse unterzieht und das 1-Hydroxyalkyl-5-nitroimidazol isoliert.

**11.** Verwendung eines Produkts der allgemeinen Formel

$$CH_3 - CO - O - (\underset{R_1}{CH})_2 - O - SO_2 - R_2$$

worin $R_2$ für einen Methylrest steht und eines der Symbole $R_1$ ein Wasserstoffatom und das andere ein Wasserstoffatom oder den Methylrest wiedergibt, erhalten nach dem Verfahren des Anspruchs 3, zur Herstellung eines 1-Hydroxyalkyl-5-nitroimidazols, dadurch gekennzeichnet, daß man es mit einem Imidazolderivat der Formel, wie in Anspruch 7 definiert, bei einer Temperatur zwischen 60 und 100°C umsetzt, wobei man gegebenenfalls in einem organischen Lösungsmittel, ausgewählt unter den Estern, wie Methyl- oder Ethylacetat, den Ethern, wie Methyl-tert.-butylether, den Ketonen, wie Methylisobutylketon, oder den aromatischen Kohlenwasserstoffen, wie Toluol oder Xylol arbeitet, danach das erhaltene Produkt einer Hydrolyse oder einer Alkoholyse unterzieht, und das 1-Hydroxyalkyl-5-nitroimidazol isoliert.

**12.** Verwendung des 2-Acetoxyethylmesylats, erhalten nach dem Verfahren des Anspruchs 6, zur Herstellung von 1-Hydroxyethyl-2-methyl-5-nitroimidazol, dadurch gekennzeichnet, daß man es mit einem Imidazolderivat der allgemeinen Formel

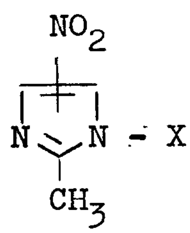

worin X für ein Wasserstoffatom, einen Hydroxyethylrest, einen Alkoxyethylrest, dessen Alkylteil 1 bis 4 Kohlenstoffatome enthält, oder einen Acyloxymethylrest, dessen Acylteil 1 bis 4 Kohlenstoffatome enthält, oder einen allylischen, ethylenischen Rest oder einen Arylmethylrest steht, bei einer Temperatur zwischen

60 und 100°C umsetzt, wobei man gegebenenfalls in einem organischen Lösungsmittel, ausgewählt unter den Estern, wie Methyl- oder Ethylacetat, den Ethern, wie Methyltert.-butylether, den Ketonen, wie Methylisobutylketon, oder den aromatischen Kohlenwasserstoffen, wie Toluol oder Xylol, arbeitet, danach das erhaltene Produkt einer Hydrolyse oder einer Alkoholyse unterwirft, und das 1-Hydroxymethyl-2-methyl-5-nitroimidazol isoliert.

## Claims

### Claims for the following Contracting States: AT, BE, CH, DE, FR, GB, IT, LI, LU, NL, SE

1. Process for the preparation of a hydroxyalkylating agent of general formula:

$$R - CO - O - (\underset{\underset{R_1}{|}}{CH})_n - O - SO_2 - R_2$$

in which
R represents an alkyl radical containing 1 to 4 carbon atoms,
n is an integer equal to 2 or 3
the symbols $R_1$, which are identical or different, represent a hydrogen atom or an alkyl radical containing 1 to 4 carbon atoms,
and
$R_2$ represents an alkyl radical containing 1 to 4 carbon atoms, phenyl or a radical

$$- O - (\underset{\underset{R_1}{|}}{CH})_n - O - CO - R$$

in which R, n and $R_1$ are defined as previously, it being understood that, in the two radicals

$$- O - (\underset{\underset{R_1}{|}}{CH})_n - O - CO - R$$

the symbols R, n and $R_1$ have the same meanings, characterised in that an acid of general formula:

$$HO - SO_2 - R_3$$

in which $R_3$ represents a hydroxyl radical or an alkyl radical containing 1 to 4 carbon atoms or phenyl is reacted with a diester of general formula:

$$R - CO - O - (\underset{\underset{R_1}{|}}{CH})_n - O - CO - R$$

in which R, n and $R_1$ are defined as above, at a temperature of between 80 and 160°C, with removal of the acid formed (RCOOH) or its methyl ester as soon as it is formed by distillation under reduced pressure of 1.3 to 26.6 kPa, and optionally of the excess of diester.

2. Process for the preparation of a hydroxyalkylating agent of general formula:

$$R - CO - O\ (\underset{\underset{R_1}{|}}{CH})_n - O - SO_2 - O - (\underset{\underset{R_1}{|}}{CH})_n - O - CO - R$$

characterised in that dimethyl sulphate is reacted with an ester of general formula:

$$R - CO - O - \underset{\substack{|\\R_1}}{(CH)_n} - O - CO - R$$

in which R, n and $R_1$ are defined as in Claim 1, at a temperature of between 80 and 160°C, with removal of the methyl ester formed ($R-CO-O-CH_3$) by distillation under reduced pressure, and optionally of the excess of diester.

3. Process for the preparation of the hydroxyalkylating agent of general formula:

$$CH_3 - CO - O - \underset{\substack{|\\R_1}}{(CH)_2} - O - SO_2 - R_2$$

in which $R_2$ represents a methyl radical or a radical

$$- O - \underset{\substack{|\\R_1}}{(CH)_2} - O - CO - CH_3$$

characterised in that sulphuric acid or methanesulphonic acid is reacted with a diester of general formula:

$$CH_3 - CO - O - \underset{\substack{|\\R_1}}{(CH)_2} - O - CO - CH_3$$

in which one of the symbols $R_1$ represents a hydrogen atom and the other represents a hydrogen atom or a methyl radical, at a temperature of between 80 and 160°C, with removal of the acetic acid formed by distillation under reduced pressure.

4. Process for the preparation of a hydroxyalkylating agent of general formula:

$$CH_3 - CO - O - \underset{\substack{|\\R_1}}{(CH)_2} - O - SO_2 - O - \underset{\substack{|\\R_1}}{(CH)_2} - O - CO - CH_3$$

characterised in that dimethyl sulphate is reacted with a diester of general formula:

$$CH_3 - CO - O - \underset{\substack{|\\R_1}}{(CH)_2} - O - CO - CH_3$$

in which one of the symbols $R_1$ represents a hydrogen atom and the other represents a hydrogen atom or a methyl radical, at a temperature of between 80 and 160°C, with removal of the methyl acetate formed by distillation under reduced pressure.

5. Process for the preparation of di(2-acetoxyethyl) sulphate, characterised in that sulphuric acid or dimethyl sulphate is reacted with glycol diacetate at a temperature of between 80 and 160°C, with removal of the acetic acid or the methyl acetate formed by distillation under reduced pressure.

6. Process for the preparation of 2-acetoxyethyl mesylate, characterised in that methanesulphonic acid is reacted with glycol diacetate at a temperature of between 80 and 160°C, with removal of the acetic acid formed by distillation under reduced pressure.

7. New hydroxyalkylating agents of general formula:

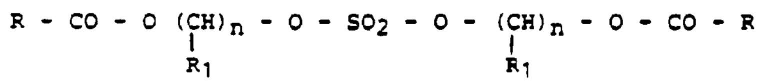

in which
R represents an alkyl radical containing 1 to 4 carbon atoms,
n is an integer equal to 2 or 3, and
the symbols $R_1$, which are identical or different, represent a hydrogen atom or an alkyl radical containing 1 to 4 carbon atoms.

**8.** New hydroxyalkylating agents of general formula:

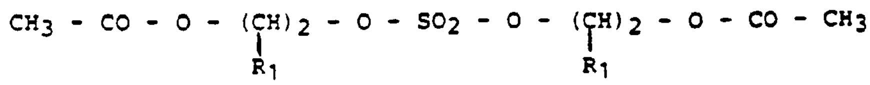

in which one of the symbols $R_1$ represents a hydrogen atom or a methyl radical.

**9.** Di(2-acetoxyethyl) sulphate.

**10.** Process for the preparation of a 1-hydroxyalkyl-5 -nitroimidazole, characterised in that a product according to Claim 7 is reacted with an imidazole derivative of general formula:

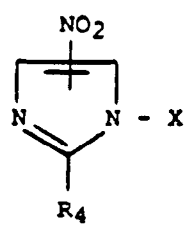

in which $R_4$ represents a hydrogen atom or an alkyl radical containing 1 to 4 carbon atoms or an alkenyl radical containing 2 to 4 carbon atoms, the alkyl and alkenyl radicals being optionally substituted by one or more radicals, which are identical or different, chosen from the phenyl, phenoxy or 5- or 6-membered, oxygenated heterocyclic radicals, or else an aryl radical containing 6 to 10 carbon atoms optionally substituted by one or more atoms or radicals, which are identical or different, chosen from the halogen atoms and alkyl containing 1 to 4 carbon atoms, alkoxy in which the alkyl part contains 1 to 4 carbon atoms, phenyl, phenoxy or nitro radicals, or else a cycloalkyl radical containing 5 or 6 carbon atoms, the phenyl, phenoxy or heterocyclic radicals being optionally substituted by one or more atoms or radicals, which are identical or different, chosen from the halogen atoms and alkyl containing 1 to 4 carbon atoms, alkoxy in which the alkyl part contains 1 to 4 carbon atoms, phenyl, phenoxy or nitro radicals and X represents a hydrogen atom or a hydroxymethyl, alkoxymethyl in which the alkyl part contains 1 to 4 carbon atoms, or acyloxymethyl, in which the acyl part contains 1 to 4 carbon atoms, radical or an allyl-like ethylenic radical or an arylmethyl radical, at a temperature of between 60 and 100°C the operation being optionally carried out in an organic solvent chosen from esters such as methyl or ethyl acetate, ethers such as methyl tert-butyl ether, ketones such as methyl isobutyl ketone or the aromatic hydrocarbons such as toluene or xylene, the product obtained is then subjected to a hydrolysis or an alcoholysis, and the 1-hydroxyalkyl-5-nitroimidazole thus obtained is isolated.

**11.** Process for the preparation of a 1-hydroxyalkyl-5-nitroimidazole, characterised in that a product according to Claim 8 is reacted with an imidazole derivative such as defined in Claim 10, at a temperature of between 60 and 100°C the operation being optionally carried out in an organic solvent chosen from esters such as methyl or ethyl acetate, ethers such as methyl tert-butyl ether, ketones such as methyl isobutyl ketone or aromatic hydrocarbons such as toluene or xylene, the product obtained is then subjected to a hydrolysis or an alcoholysis, and the 1-hydroxyalkyl-5-nitroimidazole is isolated.

**12.** Process for the preparation of 1-hydroxyethyl-2-methyl-5-nitroimidazole, characterised in that di(2-acetoxyethyl) sulphate is reacted with an imidazole derivative of general formula:

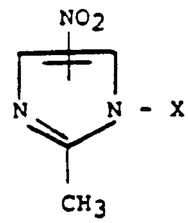

in which X represents a hydrogen atom or a hydroxymethyl, alkoxymethyl in which the alkyl part contains 1 to 4 carbon atoms, or acyloxymethyl in which the acyl part contains 1 to 4 carbon atoms, radical or an allyl-like ethylenic radical or an arylmethyl radical, at a temperature of between 60 and 100°C the operation being optionally carried out in an organic solvent chosen from esters such as methyl or ethyl acetate, ethers such as methyl tert-butyl ether, ketones such as methyl isobutyl ketone or aromatic hydrocarbons such as toluene or xylene, the product obtained is then subjected to a hydrolysis or an alcoholysis and 1-hydroxyethyl-2-methyl-5-nitroimidazole is isolated.

**13.** Process for the preparation of a 1-hydroxyalkyl-5 -nitroimidazole, characterised in that a product of general formula:

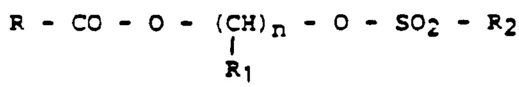

in which R represents an alkyl radical containing 1 to 4 carbon atoms, n is equal to 2 or 3, the symbols $R_1$, which are identical or different, represent a hydrogen atom or an alkyl radical containing 1 to 4 carbon atoms and $R_2$ represents an alkyl radical containing 1 to 4 carbon atoms or a phenyl radical obtained according to the process of Claim 1, is reacted with an imidazole derivative of formula such as defined in claim 10, at a temperature of between 60 and 100°C and the operation being optionally carried out in an organic solvent chosen from esters such as methyl or ethyl acetate, ethers such as methyl tert-butyl ether, ketones such as methyl isobutyl ketone or aromatic hydrocarbons such as toluene or xylene, the product obtained is then subjected to a hydrolysis or an alcoholysis, and the 1-hydroxyalkyl-5-nitroimidazole is isolated.

**14.** Process for the preparation of a 1-hydroxyalkyl-5-nitroimidazole, characterised in that a product of general formula:

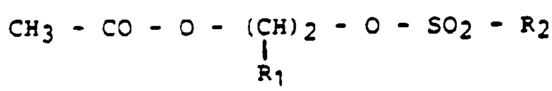

in which $R_2$ represents an methyl radical and one of the symbols $R_1$ represents a hydrogen atom and the other represents a hydrogen atom or a methyl radical obtained according to the process of Claim 3, is reacted with an imidazole derivative of formula such as defined in Claim 10, at a temperature of between 60 and 100°C and the operation being optionally carried out in an organic solvent chosen from esters such as methyl or ethyl acetate, ethers such as methyl tert-butyl ether, ketones such as methyl isobutyl ketone or aromatic hydrocarbons such as toluene or xylene, the product obtained is then subjected to a hydrolysis or an alcoholysis, and the 1-hydroxyalkyl-5-nitroimidazole is isolated.

**15.** Process for the preparation of 1-hydroxyethyl-2-methyl-5-nitroimidazole, characterised in that 2-acetoxyethyl mesylate obtained according to the process of Claim 6 is reacted with an imidazole derivative of general formula:

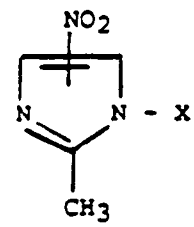

in which X represents a hydrogen atom or a hydroxymethyl, alkoxymethyl in which the alkyl part contains 1 to 4 carbon atoms or acyloxymethyl, in which the acyl part contains 1 to 4 carbon atoms, radical or an allyl-like ethylenic radical or an arylmethyl radical, at a temperature between 60 and 100°C the operation being optionally carried out in an organic solvent chosen from esters such as methyl or ethyl acetate, ethers such as methyl tert-butyl ether, ketones such as methyl isobutyl ketone or aromatic hydrocarbons such as toluene or xylene, the product obtained is then subjected to a hydrolysis or an alcoholysis, and 1-hydroxyethyl-2-methyl-5-nitroimidazole is isolated.

**Claims for the following Contracting States: ES, GR**

**1.** Process for the preparation of a hydroxyalkylating agent of general formula:

$$R - CO - O - (\underset{R_1}{CH})_n - O - SO_2 - R_2$$

in which
R represents an alkyl radical containing 1 to 4 carbon atoms,
n is an integer equal to 2 or 3
the symbols $R_1$, which are identical or different, represent a hydrogen atom or an alkyl radical containing 1 to 4 carbon atoms,
and
$R_2$ represents an alkyl radical containing 1 to 4 carbon atoms, phenyl or a radical

$$- O - (\underset{R_1}{CH})_n - O - CO - R$$

in which R, n and $R_1$ are defined as previously, it being understood that, in the two radicals

$$- O - (\underset{R_1}{CH})_n - O - CO - R$$

the symbols R, n and $R_1$ have the same meanings, characterised in that an acid of general formula:

$$HO - SO_2 - R_3$$

in which $R_3$ represents a hydroxyl radical or an alkyl radical containing 1 to 4 carbon atoms or phenyl is reacted with a diester of general formula:

$$R - CO - O - (\underset{R_1}{CH})_n - O - CO - R$$

in which R, n and $R_1$ are defined as previously, at a temperature of between 80 and 160°C, with removal of the acid formed (RCOOH) or its methyl ester as soon as it is formed by distillation under reduced pressure of 1.3 to 26.6 kPa, and optionally of the excess of diester.

**2.** Process for the preparation of a hydroxyalkylating agent of general formula:

$$R-CO-O\ \underset{\underset{R_1}{|}}{(CH)}_n-O-SO_2-O-\underset{\underset{R_1}{|}}{(CH)}_n-O-CO-R$$

characterised in that dimethyl sulphate is reacted with an ester of general formula:

$$R-CO-O-\underset{\underset{R_1}{|}}{(CH)}_n-O-CO-R$$

in which R, n and $R_1$ are defined as in Claim 1, at a temperature of between 80 and 160°C, with removal of the methyl ester formed (R-CO-O-$CH_3$) by distillation under reduced pressure, and optionally of the excess of diester.

3. Process for the preparation of a hydroxyalkylating agent of general formula:

$$CH_3-CO-O-\underset{\underset{R_1}{|}}{(CH)}_2-O-SO_2-R_2$$

in which $R_2$ represents a methyl radical or a radical

$$-O-\underset{\underset{R_1}{|}}{(CH)}_2-O-CO-CH_3$$

characterised in that sulphuric acid or methanesulphonic acid is reacted with a diester of general formula:

$$CH_3-CO-O-\underset{\underset{R_1}{|}}{(CH)}_2-O-CO-CH_3$$

in which one of the symbols $R_1$ represents a hydrogen atom and the other represents a hydrogen atom or a methyl radical, at a temperature of between 80 and 160°C, with removal of the acetic acid formed by distillation under reduced pressure.

4. Process for the preparation of a hydroxyalkylating agent of general formula:

$$CH_3-CO-O-\underset{\underset{R_1}{|}}{(CH)}_2-O-SO_2-O-\underset{\underset{R_1}{|}}{(CH)}_2-O-CO-CH_3$$

characterised in that dimethyl sulphate is reacted with the diester of general formula:

$$CH_3-CO-O-\underset{\underset{R_1}{|}}{(CH)}_2-O-CO-CH_3$$

in which one of the symbols $R_1$ represents a hydrogen atom and the other represents a hydrogen atom or a methyl radical, at a temperature of between 80 and 160°C, with removal of the methyl acetate formed by distillation under reduced pressure.

5. Process for the preparation of di(2-acetoxyethyl) sulphate, characterised in that sulphuric acid or dimethyl sulphate is reacted with glycol diacetate at a temperature of between 80 and 160°C, with removal of the acetic acid or the methyl acetate formed by distillation under reduced pressure.

6. Process for the preparation of 2-acetoxyethyl mesylate, characterised in that methanesulphonic acid is reacted with glycol diacetate at a temperature of between 80 and 160°C, with removal of the acetic acid formed by distillation under reduced pressure.

7. Use of a product of general formula:

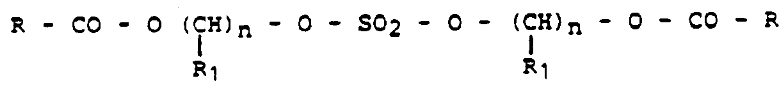

obtained according to the process of one of Claims 1 or 2 for the preparation of a 1-hydroxyalkyl-5-nitroimidazole, characterised in that it is reacted with an imidazole derivative of general formula:

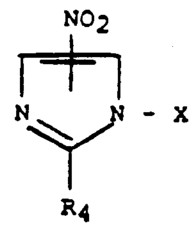

in which $R_4$ represents a hydrogen atom or an alkyl radical containing 1 to 4 carbon atoms or an alkenyl radical containing 2 to 4 carbon atoms, the alkyl and alkenyl radicals being optionally substituted by one or more radicals, which are identical or different, chosen from the phenyl, phenoxy or 5- or 6-membered, oxygenated heterocyclic radicals, or else an aryl radical containing 6 to 10 carbon atoms optionally substituted by one or more atoms or radicals, which are identical or different, chosen from the halogen atoms and alkyl containing 1 to 4 carbon atoms, alkoxy in which the alkyl part contains 1 to 4 carbon atoms, phenyl, phenoxy or nitro radicals, or else a cycloalkyl radical containing 5 or 6 carbon atoms, the phenyl, phenoxy or heterocyclic radicals being optionally substituted by one or more atoms or radicals, which are identical or different, chosen from the halogen atoms and the alkyl containing 1 to 4 carbon atoms, alkoxy in which the alkyl part contains 1 to 4 carbon atoms, phenyl, phenoxy or nitro radicals and X represents a hydrogen atom or a hydroxymethyl, alkoxymethyl in which the alkyl part contains 1 to 4 carbon atoms, or acyloxymethyl in which the acyl part contains 1 to 4 carbon atoms, radical or an allyl-like ethylenic radical or an arylmethyl radical, at a temperature of between 60 and 100°C the operation being optionally carried out in an organic solvent chosen from esters such as methyl or ethyl acetate, ethers such as methyl tert-butyl ether, ketones such as methyl isobutyl ketone or aromatic hydrocarbons such as toluene or xylene, the product obtained is then subjected to a hydrolysis or an alcoholysis, and the 1-hydroxyalkyl-5-nitroimidazole thus obtained is isolated.

8. Use of a product of general formula:

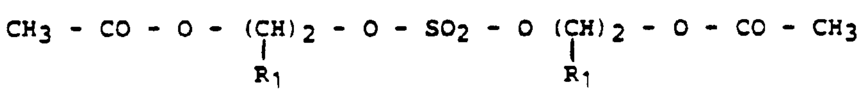

obtained according to the process of one of Claims 3 or 4 for the preparation of a 1-hydroxyalkyl-5-nitroimidazole, characterised in that it is reacted with an imidazole derivative of formula such as defined in Claim 7, at a temperature of between 60 and 100°C the operation being optionally carried out in an organic solvent chosen from esters such as methyl or ethyl acetate, ethers such as methyl tert-butyl ether, ketones such as methyl isobutyl ketone or aromatic hydrocarbons such as toluene or xylene, the product obtained is then subjected to a hydrolysis or an alcoholysis, and the 1-hydroxyalkyl-5-nitroimidazole is isolated.

9. Use of di(2-acetoxyethyl) sulphate obtained according to the process of Claim 5 for the preparation of 1-hydroxyethyl-2-methyl-5-nitroimidazole, characterised in that it is reacted with an imidazole derivative of general formula:

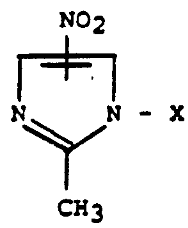

in which X represents a hydrogen atom or a hydroxymethyl, alkoxymethyl in which the alkyl part contains 1 to 4 carbon atoms, or acyloxymethyl in which the acyl part contains 1 to 4 carbon atoms, radical or an allyl-like ethylenic radical or an arylmethyl radical, at a temperature of between 60 and 100°C the operation being optionally carried out in an organic solvent chosen from esters such as methyl or ethyl acetate, ethers such as methyl tert-butyl ether, ketones such as methyl isobutyl ketone or aromatic hydrocarbons such as toluene or xylene, the product obtained is then subjected to a hydrolysis or an alcoholysis and 1-hydroxyethyl-2-methyl-5-nitroimidazole is isolated.

**10.** Use of a product of general formula:

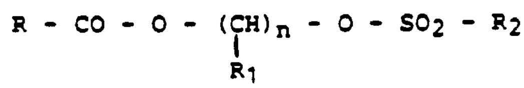

in which R represents an alkyl radical containing 1 to 4 carbon atoms, n is equal to 2 or 3, the symbols $R_1$, which are identical or different, represent a hydrogen atom or an alkyl radical containing 1 to 4 carbon atoms and $R_2$ represents an alkyl radical containing 1 to 4 carbon atoms or a phenyl radical, obtained according to the process of Claim 1 for the preparation of a 1-hydroxyalkyl-5-nitroimidazole, characterised in that it is reacted with an imidazole derivative of formula such as defined in Claim 7, at a temperature of between 60 and 100°C and the operation being optionally carried out in an organic solvent chosen from esters such as methyl or ethyl acetate, ethers such as methyl tert-butyl ether, ketones such as methyl isobutyl ketone or aromatic hydrocarbons such as toluene or xylene, the product obtained is then subjected to a hydrolysis or an alcoholysis, and the 1-hydroxyalkyl-5-nitroimidazole is isolated.

**11.** Use of a product of general formula:

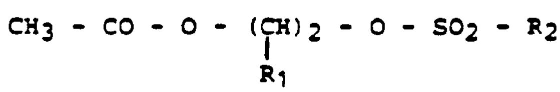

in which $R_2$ represents a methyl radical and one of the symbols $R_1$ represents a hydrogen atom and the other represents a hydrogen atom or the methyl radical, obtained according to the process of Claim 3 for the preparation of a 1-hydroxyalkyl-5-nitroimidazole, characterised in that it is reacted with an imidazole derivative of formula such as defined in Claim 7, at a temperature of between 60 and 100°C and the operation being optionally carried out in an organic solvent chosen from esters such as methyl or ethyl acetate, ethers such as methyl tert-butyl ether, ketones such as methyl isobutyl ketone or aromatic hydrocarbons such as toluene or xylene, the product obtained is then subjected to a hydrolysis or an alcoholysis, and the 1-hydroxyalkyl-5-nitroimidazole is isolated.

**12.** Use of 2-acetoxyethyl mesylate obtained according to the process of Claim 6 for the preparation of 1-hydroxyethyl-2-methyl-5-nitroimidazole, characterised in that it is reacted with an imidazole derivative of general formula:

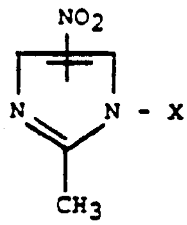

in which X represents an hydrogen atom or a hydroxymethyl, alkoxymethyl in which the alkyl part contains 1 to 4 carbon atoms or acyloxymethyl, in which the acyl part contains 1 to 4 carbon atoms, radical or an aryl-like ethylenic radical or an arylmethyl radical, at a temperature of between 60 and 100°C the operation being optionally carried out in an organic solvent chosen from esters such as methyl or ethyl acetate, ethers such as methyl tert-butyl ether, ketones such as methyl isobutyl ketone or aromatic hydrocarbons such as toluene or xylene, the product obtained is then subjected to a hydrolysis or an alcoholysis, and 1-hydroxyethyl-2-methyl-5-nitroimidazole is isolated.